# EUROPEAN PATENT SPECIFICATION

(11) **EP 2 735 284 B1**
(45) Date of publication and mention of the grant of the patent: **07.12.2016**
(21) Application number: 14155727.2
(22) Date of filing: 16.10.2009
(51) Int. Cl.: A61F 2/12, A61B 5/00, A61B 5/055

(54) **Prosthetic implant shell**
Prothesenimplantatschale
Enveloppe d'implant prothétique

(30) Priority: 17.10.2008 US 106458
(43) Date of publication of application: 28.05.2014
(62) Divisional of application: 09741147.4
(73) Proprietor: ALLERGAN, INC., Irvine, CA 92612 (US)
(72) Inventor: Yacoub, Kerim, Los Olivos, California 93441 (US); Powell, Thomas E., Santa Barbara, CA California 93111 (US)
(74) Representative: Hoffmann Eitle

(56) References cited:
- WO-A2-2010/045579
- US-A- 4 795 463
- US-A- 4 863 470

## Description

### Field of the Invention

The present invention relates to prosthetic implants, for example, mammary implants.

### Background

Implantable prostheses are commonly used to replace or augment body tissue. In the case of breast cancer, it is sometimes necessary to remove some or all of the mammary gland and surrounding tissue that creates a void that can be filled with an implantable prosthesis. The implant serves to support surrounding tissue and to maintain the appearance of the body. The restoration of the normal appearance of the body has an extremely beneficial psychological effect on post-operative patients, eliminating much of the shock and depression that often follows extensive surgical procedures. Implantable prostheses are also used more generally for restoring the normal appearance of soft tissue in various other areas of the body.

US4863740 discloses a method for producing a flexible prosthetic implant shell having an indicia. The indicia (identification marker) is made of radiopaque material composed of a matrix material (silicone) and an additive dispersed therein (bismuth trioxide or barium sulfate). The indicia is molded and shaped and then inserted within the shell.

Fluid filled implants can be imaged and evaluated in vivo using mammography, magnetic resonance imaging (MRI), ultrasonography and computer tomography (CT). MRI is one of the most accurate imaging technologies available for breast implants. However, there remains a need for technologies which afford more accurate diagnostic imaging of fluid filled implants.

### Summary

The present invention provides a method for producing a flexible prosthetic implant shell which has enhanced visibility when viewed using conventional imaging technologies, for example, magnetic resonance imaging techniques. The implant may be a mammary implant.

In one aspect of the invention, there is provided a method for producing a flexible prosthetic implant shell having an indicia, the method comprising the step of exposing a flexible implant shell comprising a matrix material and an additive dispersed therein to electromagnetic radiation such that the additive reacts with the electromagnetic radiation and forms an indicia, wherein the density of the additive is greater than that of the matrix material.

In one embodiment, the shell comprises a composition more visible to magnetic resonance imaging than conventional fluid filled implant shells. The shell comprises a matrix material, for example, an elastomeric material, such as a silicone elastomeric material, and an additive distributed in the matrix material. The additive has a density greater than the density of the matrix material. For example, the matrix material may comprise a silicone elastomer and the additive may comprise a biocompatible metal, for example, a biocompatible metal oxide dispersed throughout the silicone elastomer.

In one embodiment, the additive is a metal selected from the group of metals consisting of aluminum, brass, titanium, Nitinol (nickel-titanium alloy), steel, alloys and mixtures thereof.

In an exemplary embodiment, the additive is a metal oxide having a density of about 4.0 g/ml. More specifically, the additive may be titanium oxide (TiO₂). Even more specifically, the concentration of TiO₂ by weight is between 0.5% and 25%. In one embodiment, the concentration of TiO₂ in the shell is about 8%.

In another embodiment of the invention, the implant shell has a specific gravity of at least 1.15. For example, in some embodiments, the specific gravity of the shell is greater than 1.20 or greater than 1.40.

In one embodiment, the method comprises providing a flexible implant shell including at least one layer comprising a matrix material and a metal oxide distributed in the matrix material. In one embodiment, the metal oxide is titanium dioxide. The method further comprises providing indicia on the shell by using a laser to inscribe the indicia in the shell, wherein the metal oxide reacts with the laser radiation, for example, becomes fused, to form visible indicia in the shell. The indicia may be in the form of a label indicating batch number, manufacturer, location of manufacture, model or style number, trademark, and/or other useful information relating to the implant. In one embodiment the indicia comprises a label in the form of a bar code, for example, etched by laser on an exterior surface of the shell.

### Brief Description of the Drawing

Features and advantages of the present invention will become appreciated as the same become better understood with reference to the specification, claims, and appended drawing of which:

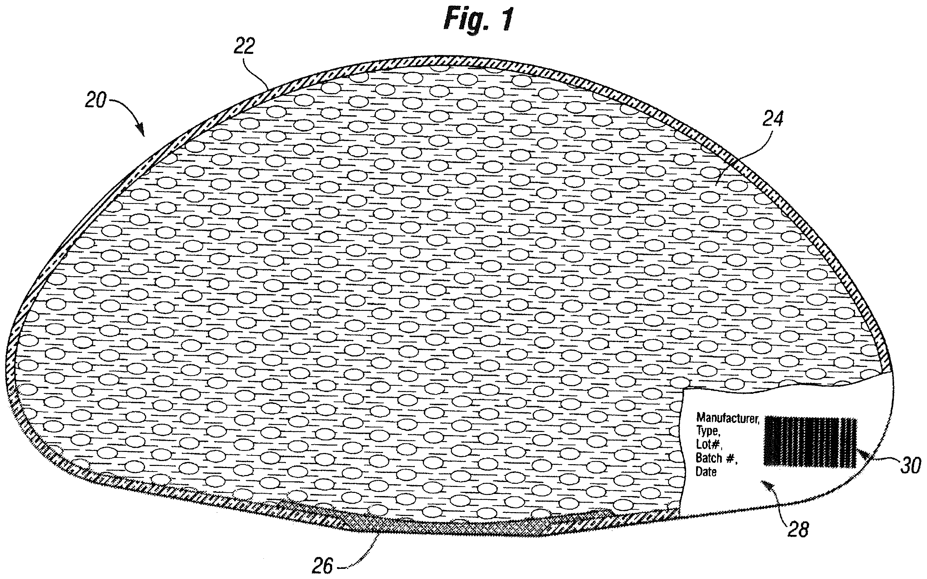

Figure 1 is a cross-sectional view through a fluid-filled prosthetic implant comprising a shell produced in accordance with an embodiment of the present invention.

### Detailed Description

The matrix material of the implant shell is typically a silicone elastomer. The additive distributed in the matrix material causes the shell to have an increased density relative to an otherwise identical shell without the additive.

The shell produced by the method of the present invention provides enhanced visibility when imaged in vivo using conventional imaging techniques, relative to conventional silicone elastomer shells not including the additive. For example, the shell and implants containing the shell are more readily visible in magnetic resonance images.

In one embodiment of the invention, the shell is made up of a material having a density greater than the density of body tissue, for example, breast tissue, adjacent the implant when the implant has been implanted in a patient.

Specific gravity is defined as the ratio of the density of a given solid or liquid substance to the density of water at 4°C (39°F). At this temperature, the density of pure water is about 1.0 g/ml (about 62.4 lb/ft³). Materials with a specific gravity greater than 1.0 have a higher density than pure water at 4°C. For practical purposes, the density of a material in g/ml (or g/cc) is equivalent to the specific gravity of that material when water is used as the reference density.

Conventional silicone elastomer implant shells have a specific gravity close to that of water, and typically no greater than about 1.10 or about 1.15. In contrast, shells of some embodiments of the present invention have a specific gravity greater than 1.15. For example, some of the implant shells in accordance with the present invention have a specific gravity greater than 1.20, greater than 1.40, greater than 1.60, or greater than 1.80.

In one embodiment, the additive comprises a non-ferromagnetic or weakly ferromagnetic material. The additive may comprise a biocompatible metal, for example, a metal oxide.

The additive may comprise, for example, a metal selected from aluminum, brass, titanium, titanium alloys, Nitinol (nickel-titanium alloy), stainless steel and blends thereof. In some embodiments, the additive is a metal oxide, specifically a non-ferromagnetic or weakly ferromagnetic metal oxide. In one embodiment, the additive is titanium dioxide (TiO₂).

The concentration of additive, for example, TiO₂, in the shell may be in a range of between 0.5% and 25% by weight, or between 5% and 10% by weight. In one embodiment, the concentration of TiO₂ in the shell is about 8% by weight.

The shell may comprise a single, unitary layer comprising the matrix material and additive as described elsewhere herein. In other embodiments, the implant shell comprises a plurality of such layers of material, wherein at least one of said layers comprises the matrix material and additive. In some embodiments of the invention, the shell has a tensile strength comparable to or greater than a tensile strength of an identical shell comprising the matrix material without said additive dispersed therein.

The additive may be added to a dispersion of the matrix material in the form of a paste or powder. The powder or paste may comprise the additive formulated with a resin, for example, a silicone fluid resin. The desired concentration of additive in the final mixture may be calculated based on percent solids of silicone elastomer (rubber) in the batch of dispersion to the amount of additive added thereto. In a specific embodiment, an additive comprising a metal oxide, for example, TiO₂, is initially provided in a powder form and then is converted to a paste form by mixing said powder with a suitable polymer or other material. The additive "paste" is then combined with a liquid form of silicone elastomer including an appropriate solvent. The combining can be accomplished by mixing or other suitable technique to ensure substantially uniform distribution of the additive in the silicone elastomer. The liquid silicone elastomer/titanium dioxide dispersion is used to form the shells of the implants of the present invention, for example, using conventional dip-molding or rotational molding techniques known to those of skill in the art.

Fig. 1 illustrates a breast implant 20 containing an exemplary shell produced by a method according to the present invention. The implant comprises a shell 22 comprising a matrix material and an additive distributed therein having a density greater than a density of the matrix material, such that the density of the shell material is greater than 1.2 g/ml or greater than 1.4 g/ml.

The implant 20 also comprises a fluid 24 enclosed by the shell 22. The fluid may be a silicone gel, saline or other appropriate prosthetic implant filler material. In some embodiments, the flush patch 26 covers a manufacturing hole formed on the shell during molding thereof. In other embodiments, the implant may include a fluid adjustment valve.

As described elsewhere herein, the shell 22 comprises the matrix material, for example, a commercially available silicone elastomer used for forming conventional implant shells, and an additive distributed therein, for example, TiO₂.

The shell 22 includes marking, labeling or other indicia 28, formed on the shell 22 by contacting the shell with focused electromagnetic energy, for example, in the form of a laser, which causes discoloration of the additive component of the shell. In one embodiment, the additive is titanium dioxide and the indicia is formed of fused titanium.

The shell comprises a matrix material and an additive which facilitates marking of the shell when the shell is contacted with radiation, for example, ultraviolet, visible, or near infrared radiation, or other radiation form which will react with the additive to form the indicia without compromising the integrity (e.g. strength) of the shell. Such energy source can be supplied by a conventional laser source. For example, in one embodiment of the invention, the additive comprises titanium dioxide and the shell includes marking, labeling or other indicia 28 of titanium, formed on the shell by reaction of focused energy with the titanium dioxide component of the shell. In one embodiment, the indicia is a computer-readable marking, for example, in the form of a bar code which provides information about the shell.

Marking of the shell may be achieved by moving a laser beam over a portion of the shell using conventional beam steering methods, by moving the shell in relation to the laser beam and/or by masking the shell and applying light energy to an unmasked portion of the shell.

Suitable lasers for use in accordance with the present invention include, for example, neodymium:yttrium aluminum garnet (Nd:YAG) lasers, carbon dioxide (CO₂) lasers, diode lasers, excimer lasers and the like.

Conventional YAG lasers emit light in the near-infrared spectrum at wavelengths of 1064 nm. Such lasers typically have continuous power outputs of from about 1 watt to about 50 watts, and can be operated in a pulsed mode at typical peak powers of from about 1 watt to about 45 kilowatts. For pulsed mode operation, frequencies of from about 1 to about 64,000 pulses/second may be used.

Suitable lasers for marking the shells of the present invention include EPILOG Legend Model 6000 L24EX-30W, EPILOG Helix Model 8000. Suitable software for use with this equipment includes CADLink Engravelab 5.0 software. Another suitable laser is Electrolox Razor 30 W razor with Scriba3 software which can interact with Oracle and generate data for the marking of the shell.

In accordance with one embodiment of the present invention, the size of the laser spot that impinges the shell may have a diameter of between 0.1 µm and 500 µm, or greater.

It will be appreciated that the laser parameters may be controlled in order to provide sufficient localized radiation to create titanium-based marking from the titanium dioxide in the shell while avoiding damage to the integrity of the shell.

In some embodiments, movement of the laser beam is controlled by a computer which may be used to create the indicia.

Alternatively or additionally, the additive may be other than titanium dioxide, for example, another suitable metal oxide, that is biocompatible and reacts with focused energy applied to the shell containing the additive to produce visible marking on the shell.

In one embodiment, the laser-inscribed indicia are detectable on the shell in vivo, for example, using MRI or other suitable imaging technique. For example, on an MRI image, the implant itself will be visible and, in addition, the indicia thereon will also be detectable, for example, distinctly visible and/or otherwise readable.

Alternatively, or in addition, the same information may be incorporated into a computer readable bar code 30 that makes automatic identification though a scanner possible. The inclusion of a non-ferromagnetic or weakly ferromagnetic metal such as TiO₂ in the shell 22 enhances the visibility of such a label, as the metal at the surface fuses to create visible lines without weakening the shell material.

## Claims

1. A method for producing a flexible prosthetic implant shell having an indicia, the method comprising the step of exposing a flexible implant shell comprising a matrix material and an additive dispersed therein to electromagnetic radiation such that the additive reacts with the electromagnetic radiation and forms an indicia, wherein the density of the additive is greater than that of the matrix material.

2. A method according to Claim 1, wherein the additive is a metal oxide.

3. A method according to Claim 2, wherein the metal oxide is TiO₂ and the indicia is formed of fused titanium.

4. A method according to any preceding claim, wherein the additive is contained in the shell in an amount of 0.5-25 wt.%.

5. A method according to Claim 4, wherein the additive is contained in the shell in an amount of 5-10 wt.%.

6. A method according to any preceding claim, wherein the electromagnetic radiation is laser radiation.

7. A method according to Claim 6, wherein the laser radiation creates a spot having a diameter of 0.1-500 µm on the shell.

8. A method according to any preceding claim, wherein the specific gravity of the shell is greater than 1.20.

9. A method according to Claim 8, wherein the specific gravity of the shell is greater than 1.40.

10. A method according to any preceding claim, wherein the indicia is in the form of a barcode.

## Patentansprüche

1. Verfahren zur Erzeugung einer flexiblen Protheseimplantatschale mit einer Identifizierung, wobei das Verfahren den Schritt der elektromagnetischen Bestrahlung einer flexiblen Implantatschale, umfassend ein Matrixmaterial und ein darin dispergiertes Additiv, enthält, so dass das Additiv mit der elektromagnetischen Strahlung reagiert und eine Identifizierung bildet, worin die Dichte des Additives größer ist als die des Matrixmaterials.

2. Verfahren nach Anspruch 1, worin das Additiv ein Metalloxid ist.

3. Verfahren nach Anspruch 2, worin das Metalloxid TiO₂ ist und die Identifizierung aus verschmolzenem Titan gebildet ist.

4. Verfahren nach einem der vorhergehenden Ansprüche, worin das Additiv in der Schale in einer Menge von 0,5-25 Gew.-% enthalten ist.

5. Verfahren nach Anspruch 4, worin das Additiv in der Schale in einer Menge von 5-10 Gew.-% enthalten ist.

6. Verfahren nach einem der vorhergehenden Ansprüche, worin die elektromagnetische Strahlung Laserstrahlung ist.

7. Verfahren nach Anspruch 6, worin die Laserstrahlung einen Punkt mit einem Durchmesser von 0,1-500 µm auf der Schale kreiert.

8. Verfahren nach einem der vorhergehenden Ansprüche, worin das spezifische Gewicht der Schale größer als 1,20 ist.

9. Verfahren nach Anspruch 8, worin das spezifische Gewicht der Schale größer als 1,40 ist.

10. Verfahren nach einem der vorhergehenden Ansprüche, worin die Identifizierung in der Form eines Barcodes vorliegt.

## Revendications

1. Procédé de production d'une enveloppe flexible d'implant prosthétique ayant un indice, le procédé comprenant l'étape d'exposition d'une enveloppe d'implant flexible comprenant un matériau de matrice et un additif dispersé dans celui-ci à un rayonnement électromagnétique de telle sorte que l'additif réagisse avec le rayonnement électromagnétique et forme un indice, dans lequel la densité de l'additif est plus élevée que celle du matériau de matrice.

2. Procédé selon la revendication 1, dans lequel l'additif est un oxyde métallique.

3. Procédé selon la revendication 2, dans lequel l'oxyde métallique est le TiO₂ et l'indice est formé de titane fondu.

4. Procédé selon l'une quelconque des revendications précédentes, dans lequel l'additif est contenu dans l'enveloppe en une quantité de 0,5 à 25 % en poids.

5. Procédé selon la revendication 4, dans lequel l'additif est contenu dans l'enveloppe en une quantité de 5 à 10 % en poids.

6. Procédé selon l'une quelconque des revendications précédentes, dans lequel le rayonnement électromagnétique est un rayon laser.

7. Procédé selon la revendication 6, dans lequel le rayon laser crée un point ayant un diamètre de 0,1 à 500 µm sur l'enveloppe.

8. Procédé selon l'une quelconque des revendications précédentes, dans lequel la densité de l'enveloppe est supérieure à 1,20.

9. Procédé selon la revendication 8, dans lequel la densité de l'enveloppe est supérieure à 1,40.

10. Procédé selon l'une quelconque des revendications précédentes, dans lequel l'indice se présente sous la forme d'un code-barres.
